(19) Europäisches Patentamt
European Patent Office
Office européen des brevets

(11) EP 4 412 427 A1

(12) **EUROPEAN PATENT APPLICATION**
published in accordance with Art. 153(4) EPC

(43) Date of publication:
**07.08.2024 Bulletin 2024/32**

(21) Application number: **22876259.7**

(22) Date of filing: **27.09.2022**

(51) International Patent Classification (IPC):
***H10K 30/50*** *(2023.01)* ***C07D 279/22*** *(2006.01)*

(52) Cooperative Patent Classification (CPC):
**C07D 279/22; H10K 30/50;** Y02E 10/549

(86) International application number:
**PCT/JP2022/036015**

(87) International publication number:
**WO 2023/054393 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2021 JP 2021159732**

(71) Applicant: **Hodogaya Chemical Co., Ltd.**
**Tokyo, 105-0021 (JP)**

(72) Inventors:
- **ITO, Toshiaki**
  **Tokyo 105-0021 (JP)**
- **TAKAHASHI, Hideaki**
  **Tokyo 105-0021 (JP)**
- **SAKURAI, Atsushi**
  **Tokyo 105-0021 (JP)**
- **SATO, Hiroshi**
  **Tokyo 105-0021 (JP)**

(74) Representative: **Müller-Boré & Partner Patentanwälte PartG mbB**
**Friedenheimer Brücke 21**
**80639 München (DE)**

# (54) COMPOUND HAVING SULFONATE GROUP, HOLE-TRANSPORTING MATERIAL, HOLE-TRANSPORTING MATERIAL COMPOSITION FOR PHOTOELECTRIC CONVERSION ELEMENT, PHOTOELECTRIC CONVERSION ELEMENT AND SOLAR CELL

(57) A compound represented by the following Formula (1), a hole transport material containing the compound, a hole transport material composition for a photoelectric conversion element containing the hole transport material, a photoelectric conversion element using the hole transport material composition for a photoelectric conversion element, and a solar cell using the photoelectric conversion element.

[Chem. 1]

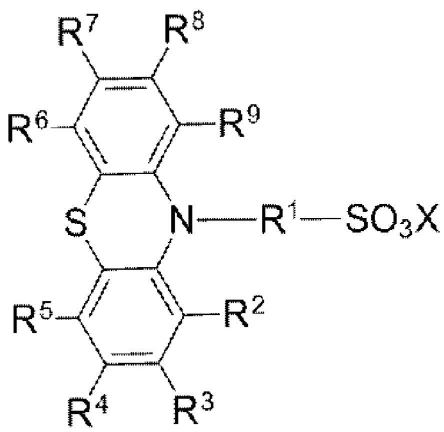

(1)

EP 4 412 427 A1

# FIGURE

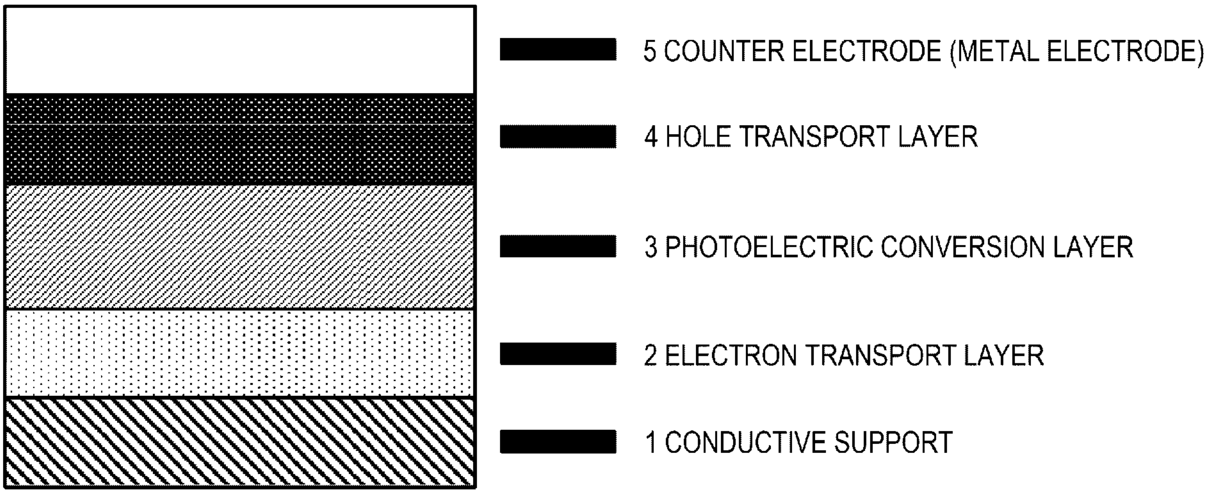
5 COUNTER ELECTRODE (METAL ELECTRODE)
4 HOLE TRANSPORT LAYER
3 PHOTOELECTRIC CONVERSION LAYER
2 ELECTRON TRANSPORT LAYER
1 CONDUCTIVE SUPPORT

## Description

### TECHNICAL FIELD

The present invention relates to a compound having a sulfonate group, a hole transport material containing the compound, a hole transport material composition for a photoelectric conversion element containing the hole transport material, a photoelectric conversion element using the hole transport material composition for a photoelectric conversion element, and a solar cell using the photoelectric conversion element.

### BACKGROUND ART

In recent years, photovoltaics has attracted attention as clean energy, and solar cells have been actively developed. The development of a solar cell using a perovskite material as a photoelectric conversion layer (hereinafter, referred to as the perovskite solar cell) is attracting attention as a next-generation solar cell that can be manufactured at low cost by a solution process (for example, Patent Literature 1, and Non Patent Literatures 1 to 2).

The perovskite solar cell often uses a hole transport material in an element. The objects to use the hole transport material include (1) improving a photoelectric conversion efficiency and (2) protecting a perovskite material that is susceptible to moisture and oxygen (for example, Non Patent Literature 3). Spiro-OMeTAD is often used as a standard hole transport material, and there are few reports of the hole transport material that contributes to photoelectric conversion characteristics more than Spiro-OMeTAD.

When a hole transport material is used in an element of the perovskite solar cell, in a method in the related art for producing an element using an organic compound as the hole transport material, a dopant, which is an additive, is added to the hole transport material. The dopant is used to reduce an electrical resistance of the hole transport material (for example, Non Patent Literatures 3 to 4).

### CITATION LIST

### PATENT LITERATURE

Patent Literature 1: US10937972B

### NON PATENT LITERATURE

Non Patent Literature 1: Journal of the American Chemical Society, 2009, Volume 131, P. 6050 to 6051
Non Patent Literature 2: Science, 2012, Volume 388, P. 643 to 647
Non Patent Literature 3: Chem. Sci., 2019, 10, P. 6748 to 6769
Non Patent Literature 4: Adv. Funct. Mater., 2019, 1901296

### SUMMARY OF INVENTION

### TECHNICAL PROBLEM

The problem to be solved by the present invention is to provide a compound that is useful as a hole transport material for a photoelectric conversion element, and a photoelectric conversion element and a solar cell that use the compound in a hole transport layer for a photoelectric conversion element and have good photoelectric conversion characteristics.

### SOLUTION TO PROBLEM

In order to solve the above problem, as a result of intensive studies on improving photoelectric conversion characteristics, the inventors have found that the use of a compound having a specific structure as a hole transport layer provides a solar cell and a photoelectric conversion element with good photoelectric conversion efficiency and high durability. That is, the gist of the present invention is as follows.

1. A compound represented by the following Formula (1).

[Chem. 1]

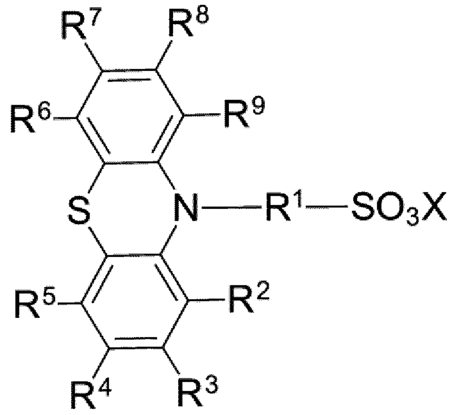

(1)

[In the formula, $R^1$ represents: a linear or branched alkylene group having 1 to 18 carbon atoms that optionally has a substituent; a linear or branched alkenylene group having 2 to 20 carbon atoms that optionally has a substituent; a linear or branched alkynylene group having 2 to 20 carbon atoms that optionally has a substituent; a cycloalkylene group having 3 to 10 carbon atoms that optionally has a substituent; an arylene group having 6 to 36 carbon atoms that optionally has a substituent; or a divalent heterocyclic group having 5 to 36 ring atoms that optionally has a substituent, and X represents a monovalent cation. $R^2$ to $R^9$ each independently represent: a hydrogen atom; a linear or branched alkyl group having 1 to 18 carbon atoms that optionally has a substituent; a linear or branched alkenyl group having 2 to 20 carbon atoms that optionally has a substituent; a linear or branched alkynyl group having 2 to 20 carbon atoms that optionally has a substituent; a cycloalkyl group having 3 to 10 carbon atoms that optionally has a substituent; a linear or branched alkoxy group having 1 to 20 carbon atoms that optionally has a substituent; a cycloalkoxy group having 3 to 10 carbon atoms that optionally has a substituent; a linear or branched alkoxycarbonyl group having 1 to 18 carbon atoms that optionally has a substituent; a thio group having 1 to 18 carbon atoms that optionally has a substituent; an amino group having 1 to 20 carbon atoms that optionally has a substituent; an aromatic hydrocarbon group having 6 to 36 carbon atoms that optionally has a substituent; or a monovalent heterocyclic group having 5 to 36 ring atoms that optionally has a substituent].

2. The compound, in which in Formula (1), $R^1$ represents the linear or branched alkylene group having 1 to 18 carbon atoms that optionally has a substituent.

3. The compound, in which in Formula (1), at least one of $R^2$ to $R^9$ represents the aromatic hydrocarbon group having 6 to 36 carbon atoms that optionally has a substituent or the amino group having 1 to 20 carbon atoms that optionally has a substituent.

4. The compound, in which in Formula (1), at least one of $R^4$ and $R^7$ represents the aromatic hydrocarbon group having 6 to 36 carbon atoms that optionally has a substituent or the amino group having 1 to 20 carbon atoms that optionally has a substituent. 5. The compound, in which in Formula (1), X represents an alkali metal ion or an ammonium ion that optionally has a substituent.

6. The compound, in which in Formula (1), the alkali metal ion includes a sodium ion, a potassium ion, a rubidium ion, and a cesium ion.

7. A hole transport material containing the compound described above.

8. A hole transport material composition for a photoelectric conversion element containing the hole transport material described above.

9. A photoelectric conversion element using the hole transport material composition for photoelectric conversion described above.

10. A solar cell using the photoelectric conversion element described above.

ADVANTAGEOUS EFFECTS OF INVENTION

A compound having a sulfonate group and a hole transport layer using the compound according to the present invention allows for providing a solar cell and a photoelectric conversion element with good photoelectric conversion efficiency and high durability by using the compound between the photoelectric conversion layer and an electrode.

BRIEF DESCRIPTION OF DRAWINGS

The FIGURE is a schematic cross-sectional view showing a configuration of a photoelectric conversion element according to an example and a comparative example of the present invention.

DESCRIPTION OF EMBODIMENTS

Hereinafter, embodiments of the present invention will be described in detail. A hole transport layer of the present invention is used in a photoelectric conversion element and a perovskite photoelectric conversion element.

The compound represented by Formula (1) is specifically described below, but the present invention is not limited thereto.

In Formula (1), $R^1$ represents: a linear or branched alkylene group having 1 to 18 carbon atoms that optionally has a substituent; a linear or branched alkenylene group having 2 to 20 carbon atoms that optionally has a substituent; a linear or branched alkynylene group having 2 to 20 carbon atoms that optionally has a substituent; a cycloalkylene group having 3 to 10 carbon atoms that optionally has a substituent; an arylene group having 6 to 36 carbon atoms that optionally has a substituent; or a divalent heterocyclic group having 5 to 36 ring atoms that optionally has a substituent.

In Formula (1), specific examples of the "linear or branched alkylene group having 1 to 18 carbon atoms" in the "linear or branched alkylene group having 1 to 18 carbon atoms that optionally has a substituent" represented by $R^1$ may include an alkylene group obtained by removing one hydrogen from an alkyl group such as a methyl group, an ethyl group, an n-propyl group, an isopropyl group, an n-butyl group, an isobutyl group, an s-butyl group, a t-butyl group, an n-pentyl group, an isopentyl group, an n-hexyl group, a 2-ethylhexyl group, a heptyl group, an octyl group, an isooctyl group, a nonyl group, and a decyl group. One of hydrogens in the alkyl groups listed above or a substituent is substituted with a sulfonate group ($-SO_3X$) in Formula (1).

In Formula (1), specific examples of the "linear or branched alkenyl group having 2 to 20 carbon atoms" in the "linear or branched alkenylene group having 2 to 20 carbon atoms that optionally has a substituent" represented by $R^1$ may include an alkenylene group obtained by removing one hydrogen from a vinyl group, a 1-propenyl group, an allyl group, a 1-butenyl group, a 2-butenyl group, a 1-pentenyl group, a 1-hexenyl group, an isopropenyl group, an isobutenyl group, or a linear or branched alkenyl group having 2 to 20 carbon atoms in which a plurality of these alkenyl groups are bonded. One of hydrogens in the alkenyl groups listed above or a substituent is substituted with a sulfonate group ($-SO_3X$) in Formula (1).

In Formula (1), specific examples of the "linear or branched alkynylene group having 2 to 20 carbon atoms" in the "linear or branched alkynylene group having 2 to 20 carbon atoms that optionally has a substituent" represented by $R^1$ may include an alkynylene group obtained by removing one hydrogen from an alkynyl group such as an ethynyl group, a 1-propynyl group, a 2-propynyl group, a 1-butynyl group, a 2-butynyl group, a 1-methyl-2-propynyl group, a 1-pentynyl group, a 2-pentynyl group, a 1-methyl-n-butynyl group, a 2-methyl-n-butynyl group, a 3-methyl-n-butynyl group, and a 1-hexynyl group. One of hydrogens in the alkynyl groups listed above or a substituent is substituted with a sulfonate group ($-SO_3X$) in Formula (1).

In Formula (1), specific examples of the "cycloalkyl group having 3 to 10 carbon atoms" in the "cycloalkylene group having 3 to 10 carbon atoms that optionally has a substituent" represented by $R^1$ may include a cycloalkylene group obtained by removing one hydrogen from a cycloalkyl group such as a cyclopropyl group, a cyclobutyl group, a cyclopentyl group, a cyclohexyl group, a cycloheptyl group, a cyclooctyl group, a cyclodecyl group, and a cyclododecyl group. One of hydrogens in the cycloalkyl groups listed above or a substituent is substituted with a sulfonate group ($-SO_3X$) in Formula (1).

In Formula (1), specific examples of the "arylene group having 6 to 36 carbon atoms" in the "arylene group having 6 to 36 carbon atoms that optionally has a substituent" represented by $R^1$ may include an arylene group obtained by removing one hydrogen from an aromatic hydrocarbon group such as a phenyl group, a biphenyl group, a terphenyl group, a naphthyl group, a biphenyl group, an anthracenyl group (anthryl group), a phenanthryl group, a fluorenyl group, an indenyl group, a pyrenyl group, a perylenyl group, a fluoranthenyl group, and a triphenylenyl group. One of hydrogens in the aromatic hydrocarbon groups listed above or a substituent is substituted with a sulfonate group ($-SO_3X$) in Formula (1). In the present invention, the aromatic hydrocarbon group includes a "condensed polycyclic aromatic group".

In Formula (1), specific examples of the "heterocyclic group having 5 to 36 ring atoms" in the "divalent heterocyclic group having 5 to 36 ring atoms that optionally has a substituent" represented by $R^1$ may include a divalent heterocyclic group obtained by removing one hydrogen from a monovalent heterocyclic group such as a pyridyl group, a pyrimidylinyl group, a triazinyl group, a thienyl group, a furyl group (furanyl group), a pyrrolyl group, an imidazolyl group, a pyrazolyl group, a triazolyl group, a quinolyl group, an isoquinolyl group, a naphthyldinyl group, an acridinyl group, a phenanthrolinyl group, a benzofuranyl group, a benzothienyl group, an oxazolyl group, an indolyl group, a carbazolyl group, a benzoxazolyl group, a thiazolyl group, benzothiazolyl group, a quinoxalinyl group, a benzimidazolyl group, a pyrazolyl group, a dibenzofuranyl group, a dibenzothienyl group, and a carbonylyl group. One of hydrogens in the monovalent heterocyclic

groups listed above or a substituent is substituted with a sulfonate group ($-SO_3X$) in Formula (1).

In Formula (1), specific examples of the "substituent" in the "linear or branched alkylene group having 1 to 18 carbon atoms that optionally has a substituent", the "linear or branched alkenylene group having 2 to 20 carbon atoms that optionally has a substituent", the "linear or branched alkynylene group having 2 to 20 carbon atoms that optionally has a substituent", the "cycloalkylene group having 3 to 10 carbon atoms that optionally has a substituent", the "arylene group having 6 to 36 carbon atoms that optionally has a substituent", or the "divalent heterocyclic group having 5 to 36 ring atoms that optionally has a substituent" represented by $R^1$ may include:

a halogen atom such as a fluorine atom, a chlorine atom, a bromine atom, and an iodine atom; a cyano group; a hydroxyl group; a nitro group; a nitroso group; a carboxyl group; a phosphate group;
a carboxylic acid ester group such as a methyl ester group and an ethyl ester group;
a linear or branched alkyl group having 1 to 18 carbon atoms such as a methyl group, an ethyl group, an n-propyl group, an isopropyl group, an n-butyl group, an isobutyl group, an s-butyl group, a t-butyl group, an n-pentyl group, an isopentyl group, an n-hexyl group, a 2-ethylhexyl group, a heptyl group, an octyl group, an isooctyl group, a nonyl group, and a decyl group;
a linear or branched alkenyl group having 2 to 20 carbon atoms such as a vinyl group, a 1-propenyl group, an allyl group, a 1-butenyl group, a 2-butenyl group, a 1-pentenyl group, a 1-hexenyl group, an isopropenyl group, and an isobutenyl group;
a linear or branched alkoxy group having 1 to 18 carbon atoms such as a methoxy group, an ethoxy group, a propoxy group, a t-butoxy group, a pentyloxy group, and a hexyloxy group;
an aromatic hydrocarbon group having 6 to 30 carbon atoms such as a phenyl group, a naphthyl group, an anthryl group, a phenanthryl group, and a pyrenyl group;
a heterocyclic group having 5 to 30 ring atoms such as a pyridyl group, a pyrimidylinyl group, a triazinyl group, a thienyl group, a furyl group (furanyl group), a pyrrolyl group, an imidazolyl group, a pyrazolyl group, a triazolyl group, a quinolyl group, a isoquinolyl group, a naphthyldinyl group, an acridinyl group, a phenanthrolinyl group, a benzofuranyl group, a benzothienyl group, an oxazolyl group, an indolyl group, a carbazolyl group, a benzoxazolyl group, a thiazolyl group, benzothiazolyl group, a quinoxalinyl group, a benzimidazolyl group, a pyrazolyl group, a dibenzofuranyl group, a dibenzothienyl group, and a carbonylyl group;
an amino group having 0 to 18 carbon atoms such as an unsubstituted amino group ($-NH_2$), a monosubstituted amino group such as an ethylamino group, an acetylamino group, and a phenylamino group, and a disubstituted amino group such as a diethylamino group, a diphenylamino group, and an acetylphenylamino group; and
a thio group having 0 to 18 carbon atoms such as an unsubstituted thio group (thiol group: -SH), a methylthio group, an ethylthio group, a propylthio group, a hex-5-ene-3-thio group, a phenylthio group, and a biphenylthio group. Only one of the "substituents" may be included, or two or more "substituents" may be included in the above groups. When the two or more "substituents" are included, the substituents may be the same or different. The "substituents" may further have the substituents exemplified above.

X in the sulfonate group ($-SO_3X$) in Formula (1) represents a monovalent cation. Specifically, the monovalent cation is preferably an alkali metal ion, an ammonium ion that optionally has a substituent, or a phosphonium ion that optionally has a substituent, but is not limited thereto.

Specific examples of the alkali metal ion include a lithium ion, a sodium ion, a potassium ion, a rubidium ion, a cesium ion, and a francium ion, and the alkali metal ion is preferably the sodium ion, the potassium ion, the rubidium ion, and the cesium ion.

Specific examples of the ammonium ion that optionally has a substituent group include a methylammonium ion, a methylammonium monofluoride ion, a methylammonium difluoride ion, a methylammonium trifluoride ion, an ethylammonium ion, an isopropylammonium ion, an n-propylammonium ion, an isobutylammonium ion, an n-butylammonium ion, a t-butylammonium ion, a dimethylammonium ion, a diethylammonium ion, a phenylammonium ion, a benzylammonium ion, a phenethylammonium ion, a guanidinium ion, a formamidinium ion, an acetamidinium ion, an imidazolium ion, a tri-n-butylammonium ion, and a tetra-n-butylammonium ion.

Examples of the phosphonium ion that optionally has a substituent include one obtained by replacing a nitrogen atom of the ammonium ion with a phosphorus atom, such as an ethyl phosphonium ion, an isopropyl phosphonium ion, an n-propyl phosphonium ion, an isobutylaphosphonium ion, an n-butyl phosphonium ion, a t-butyl phosphonium ion, a dimethyl phosphonium ion, a diethyl phosphonium ion, a phenyl phosphonium ion, and a benzyl phosphonium ion.

In Formula (1), $R^1$ preferably represents the linear or branched alkylene group having 1 to 18 carbon atoms that optionally has a substituent.

In Formula (1), $R^2$ to $R^9$ each independently represent: a hydrogen atom; a linear or branched alkyl group having 1 to 18 carbon atoms that optionally has a substituent; a linear or branched alkenyl group having 2 to 20 carbon atoms that optionally has a substituent; a linear or branched alkynyl group having 2 to 20 carbon atoms that optionally

has a substituent; a cycloalkyl group having 3 to 10 carbon atoms that optionally has a substituent; a linear or branched alkoxy group having 1 to 20 carbon atoms that optionally has a substituent; a cycloalkoxy group having 3 to 10 carbon atoms that optionally has a substituent; a linear or branched alkoxycarbonyl group having 1 to 18 carbon atoms that optionally has a substituent; a thio group having 1 to 18 carbon atoms that optionally has a substituent; an amino group having 1 to 20 carbon atoms that optionally has a substituent; an aromatic hydrocarbon group having 6 to 36 carbon atoms that optionally has a substituent; or a monovalent heterocyclic group having 5 to 36 ring atoms that optionally has a substituent.

In Formula (1), examples of the "linear or branched alkyl group having 1 to 18 carbon atoms" in the "linear or branched alkyl group having 1 to 18 carbon atoms that optionally has a substituent" represented by $R^2$ to $R^9$ may include groups that are the same as the alkyl groups before one hydrogen is removed, which are described in the "linear or branched alkylene group having 1 to 18 carbon atoms that optionally has a substituent" represented by $R^1$ in Formula (1).

In Formula (1), examples of the "linear or branched alkenyl group having 2 to 20 carbon atoms" in the "linear or branched alkenyl group having 2 to 20 carbon atoms that optionally has a substituent" represented by $R^2$ to $R^9$ may include groups that are the same as the alkenyl groups before one hydrogen is removed, which are described in the "linear or branched alkenylene group having 2 to 20 carbon atoms" represented by $R^1$ in Formula (1).

In Formula (1), examples of the "linear or branched alkynyl group having 2 to 20 carbon atoms" in the "linear or branched alkynyl group having 2 to 20 carbon atoms that optionally has a substituent" represented by $R^2$ to $R^9$ may include groups that are the same as the alkynyl groups before one hydrogen is removed, which are described in the "linear or branched alkynylene group having 2 to 20 carbon atoms" represented by $R^1$ in Formula (1).

In Formula (1), examples of the "cycloalkyl group having 3 to 10 carbon atoms" in the "cycloalkyl group having 3 to 10 carbon atoms that optionally has a substituent" represented by $R^2$ to $R^9$ may include groups that are the same as the cycloalkyl groups before one hydrogen is removed, which are described in the "cycloalkylene group having 3 to 10 carbon atoms" represented by $R^1$ in Formula (1).

In Formula (1), specific examples of the "linear or branched alkoxy group having 1 to 20 carbon atoms" in the "linear or branched alkoxy group having 1 to 20 carbon atoms that optionally has a substituent group" represented by $R^2$ to $R^9$ may include a methoxy group, an ethoxy group, a propoxy group, an n-butoxy group, an n-pentyloxy group, n-hexyloxy group, a heptyloxy group, an octyloxy group, a nonyloxy group, a decyloxy group, an isopropoxy group, an isobutoxy group, an s-butoxy group, t-butoxy group, an isooctyloxy group, a t-octyloxy group, a phenoxy group, a tolyloxy group, a biphenylyloxy group, a terphenylyloxy group, a naphthyloxy group, an anthryloxy group, a phenanthryloxy group, a fluorenyloxy group, and an indenyloxy group.

In Formula (1), specific examples of the "linear or branched cycloalkoxy group having 3 to 10 carbon atoms" in the "linear or branched cycloalkoxy group having 3 to 10 carbon atoms that optionally has a substituent group" represented by $R^2$ to $R^9$ may include a cyclopropoxy group, a cyclobutoxy group, a cyclopentyloxy group, and a cyclohexyloxy group.

In Formula (1), specific examples of the "linear or branched alkoxycarbonyl group having 1 to 18 carbon atoms" in the "linear or branched alkoxycarbonyl group having 1 to 18 carbon atoms that optionally has a substituent group" represented by $R^2$ to $R^9$ may include a methoxycarbonyl group and an ethoxycarbonyl group.

In Formula (1), specific examples of the "thio group having 1 to 18 carbon atoms" in the "thio group having 1 to 18 carbon atoms that optionally has a substituent group" represented by $R^2$ to $R^9$ may include a methylthio group, an ethylthio group, a propylthio group, a phenylthio group, and a biphenylthio group.

In Formula (1), specific examples of the "amino group having 1 to 20 carbon atoms" in the "amino group having 1 to 20 carbon atoms that optionally has a substituent group" represented by $R^2$ to $R^9$ may include a monosubstituted amino group such as an ethylamino group, an acetylamino group, and a phenylamino group, and a disubstituted amino group such as a diethylamino group, a diphenylamino group, and an acetylphenylamino group.

InFormula (1), examples of the "aromatic hydrocarbon group having 6 to 36 carbon atoms" in the "aromatic hydrocarbon group having 6 to 36 carbon atoms that optionally has a substituent" represented by $R^2$ to $R^9$ may include groups that are the same as the aromatic hydrocarbon groups before one hydrogen is removed, which are described in the "arylene group having 6 to 36 carbon atoms that optionally has a substituent" represented by $R^1$ in Formula (1).

In Formula (1), examples of the "heterocyclic group having 5 to 36 ring atoms" in the "monovalent heterocyclic group having 5 to 36 ring atoms that optionally has a substituent" represented by $R^2$ to $R^9$ may include groups that are the same as the monovalent heterocyclic groups before one hydrogen is removed, which are described in the "divalent heterocyclic group having 5 to 36 ring atoms that optionally has a substituent" represented by $R^1$ in Formula (1).

In Formula (1), examples of the "substituent" in the "linear or branched alkyl group having 1 to 18 carbon atoms that optionally has a substituent", the "linear or branched alkenyl group having 2 to 20 carbon atoms that optionally has a substituent", the "linear or branched alkynyl group having 2 to 20 carbon atoms that optionally has a substituent", the "cycloalkyl group having 3 to 10 carbon atoms that optionally has a substituent", the "linear or branched alkoxy group having 1 to 20 carbon atoms that optionally has a substituent", the "cycloalkoxy group having 3 to 10 carbon atoms that optionally has a substituent", the "linear or branched alkoxycarbonyl group having 1 to 18 carbon atoms that optionally

has a substituent", the "thio group having 1 to 18 carbon atoms that optionally has a substituent", the "amino group having 1 to 20 carbon atoms that optionally has a substituent", the "aromatic hydrocarbon group having 6 to 36 carbon atoms that optionally has a substituent", or the "heterocyclic group having 5 to 36 ring atoms that optionally has a substituent" represented by $R^2$ to $R^9$ may include groups that are the same as the "substituents" of the "linear or branched alkylene group having 1 to 18 carbon atoms that optionally has a substituent" and the like represented by $R^1$ in Formula (1).

In Formula (1), at least one of $R^2$ to $R^9$ preferably represents the aromatic hydrocarbon group having 6 to 36 carbon atoms that optionally has a substituent or the amino group having 1 to 20 carbon atoms that optionally has a substituent.

At least one of $R^3$, $R^4$, $R^7$, and $R^8$ more preferably represents the aromatic hydrocarbon group having 6 to 36 carbon atoms that optionally has a substituent or the amino group having 1 to 20 carbon atoms that optionally has a substituent.

At least one of $R^4$ and $R^7$ even more preferably represents the aromatic hydrocarbon group having 6 to 36 carbon atoms that optionally has a substituent or the amino group having 1 to 20 carbon atoms that optionally has a substituent.

Specific examples of the compound represented by Formula (1) of the present invention are shown below, but the present invention is not limited thereto. In addition, the following exemplified compounds are described with some hydrogen atoms, carbon atoms, and the like omitted, and are examples of possible isomers, and include all other isomers. Each compound may be a mixture of two or more isomers.

[Chem. 2]

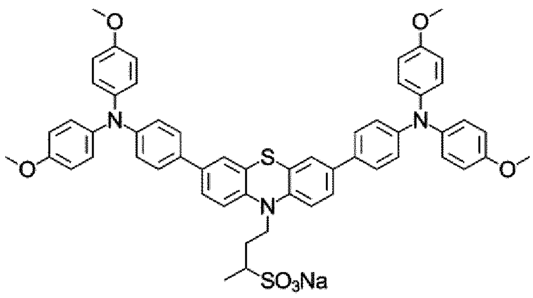

(A−1)

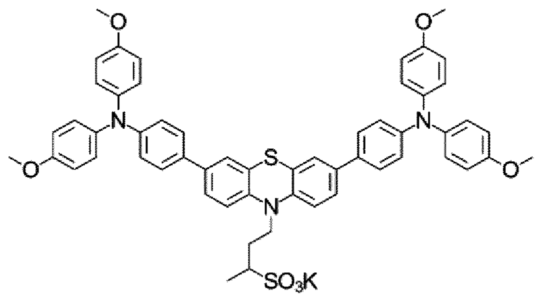

(A−2)

[Chem. 3]

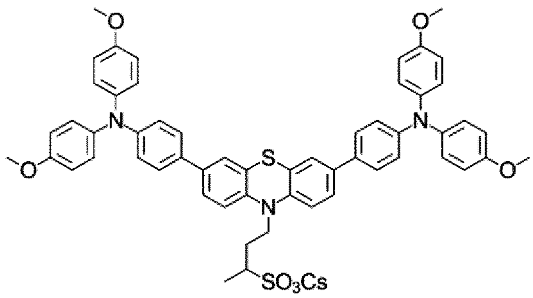

(A−3)

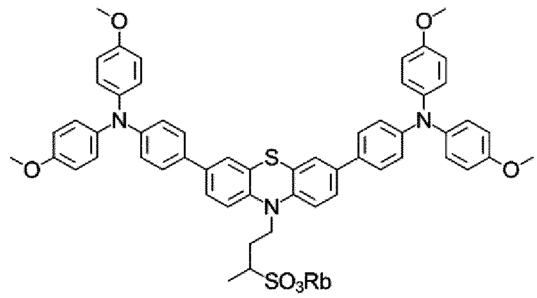

(A−4)

[Chem. 4]

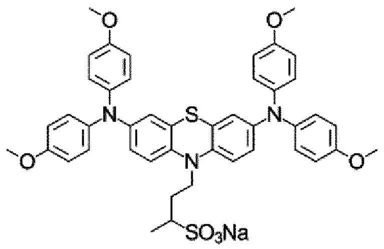

(A-5)

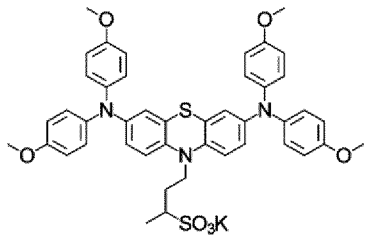

(A-6)

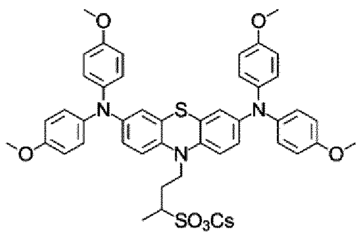

(A-7)

[Chem. 5]

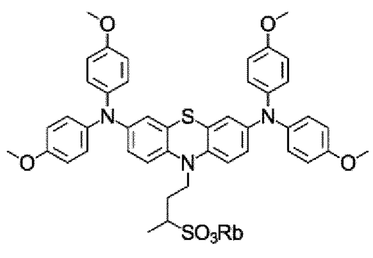

(A-8)

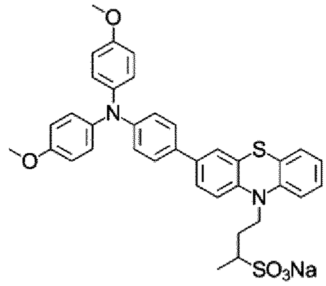

(A-9)

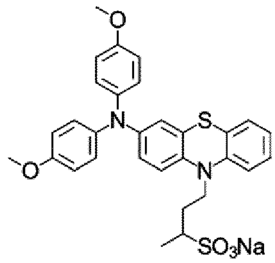

(A-10)

[Chem. 6]

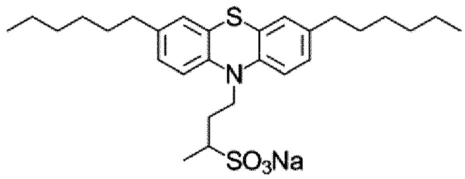

(A-11)

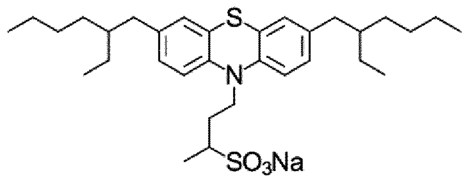

(A-12)

[Chem. 7]

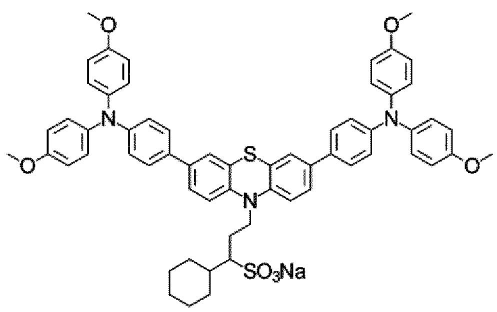

(A-13)

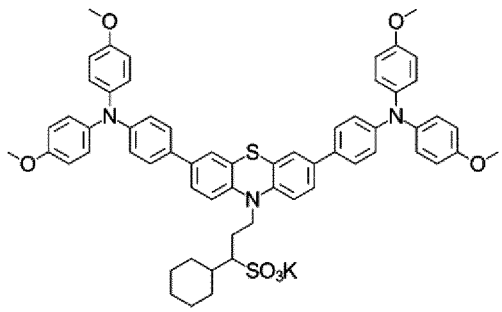

(A-14)

[Chem. 8]

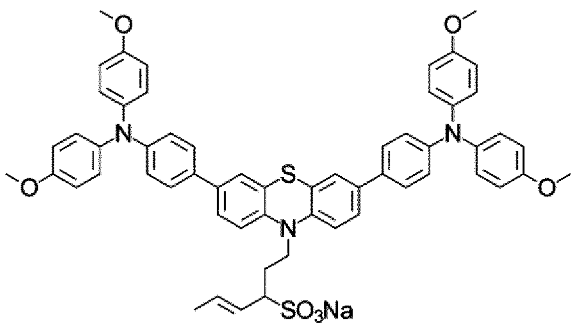

(A−15)

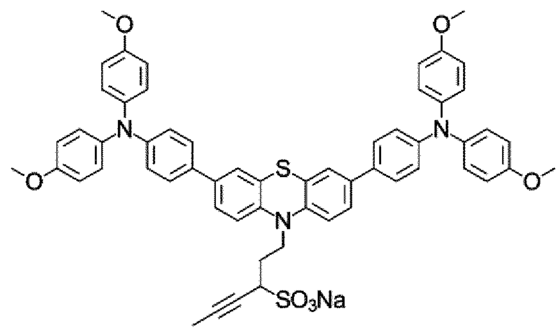

(A−16)

[Chem. 9]

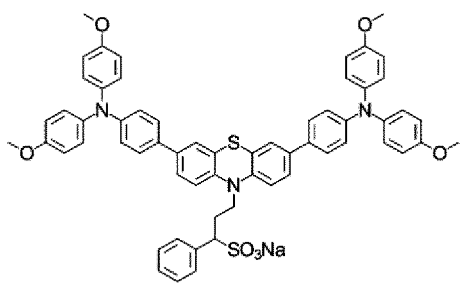

(A−17)

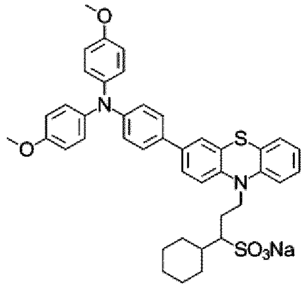

(A−18)

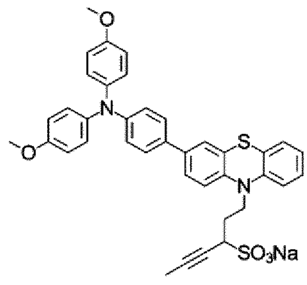

(A−19)

[Chem. 10]

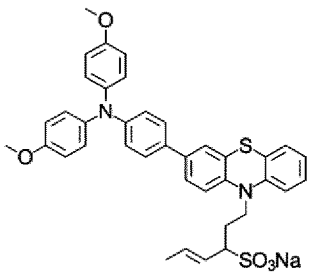

(A−20)

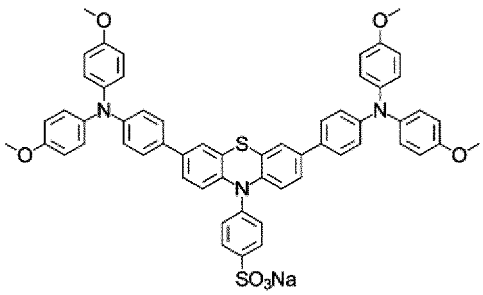

(A−21)

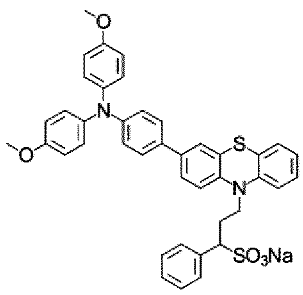

(A−22)

[Chem. 11]

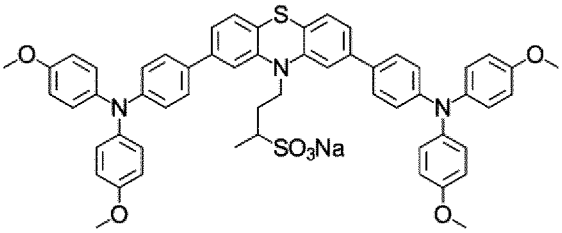

(A−23)

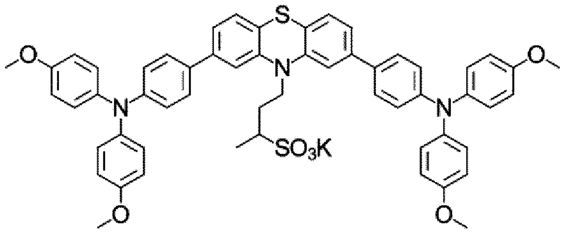

(A−24)

[Chem. 12]

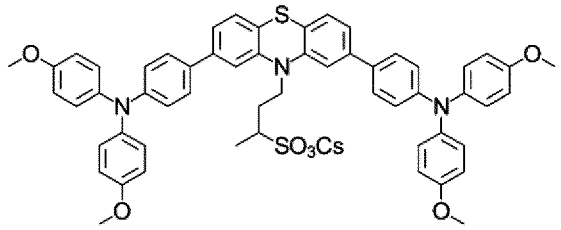

(A-25)

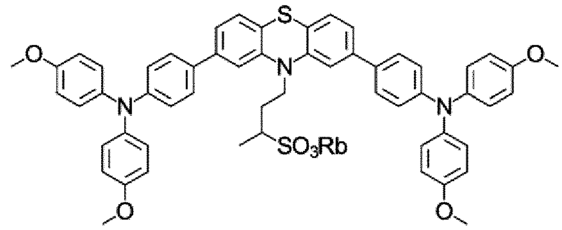

(A-26)

[Chem. 13]

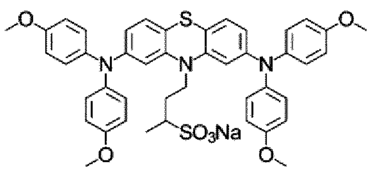

(A-27)

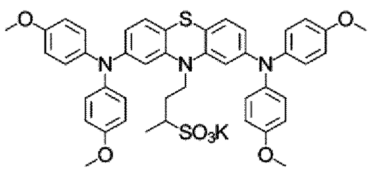

(A-28)

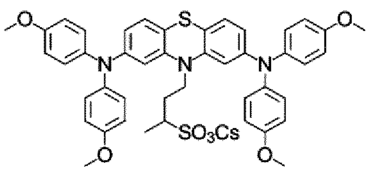

(A-29)

[Chem. 14]

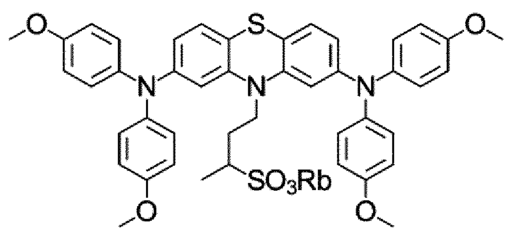

(A-30)

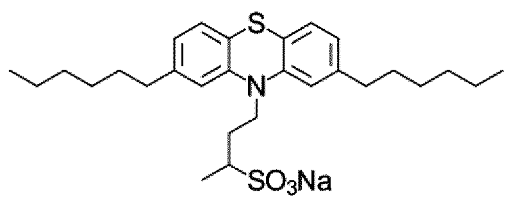

(A-31)

[Chem. 15]

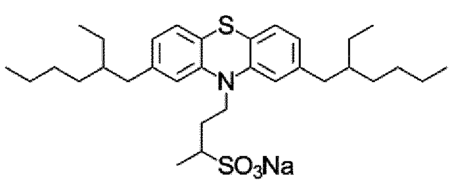

(A-32)

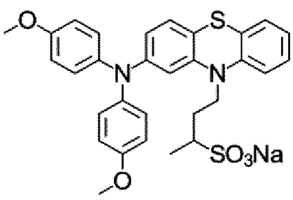

(A-33)

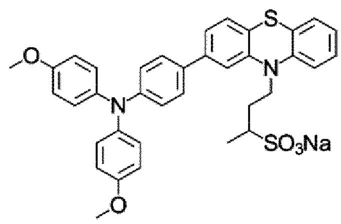

(A-34)

[Chem. 16]

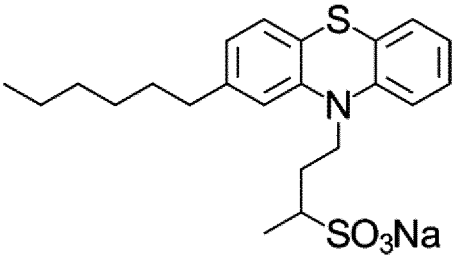

(A－35)

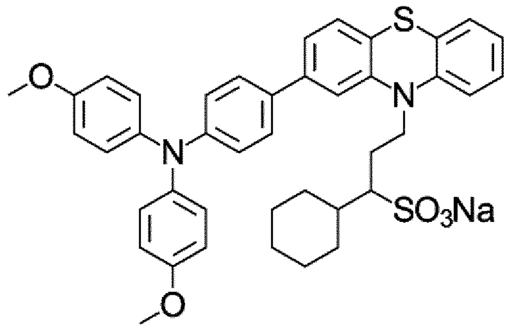

(A－36)

[Chem. 17]

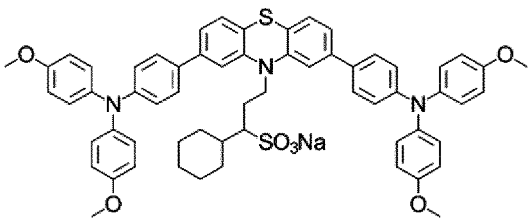

(A－37)

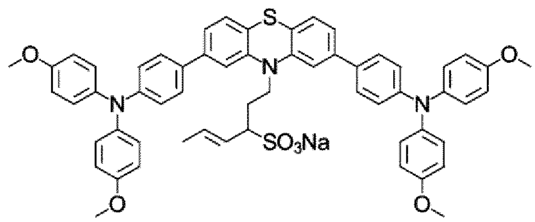

(A－38)

[Chem. 18]

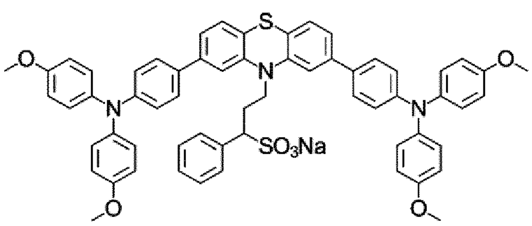

(A－39)

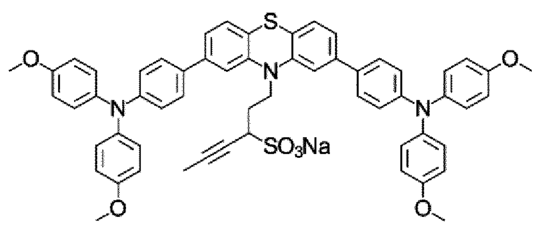

(A－40)

[Chem. 19]

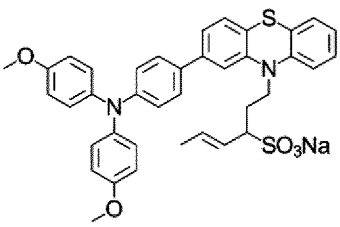

(A－41)

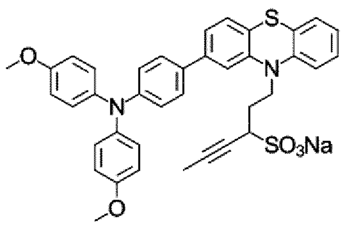

(A－42)

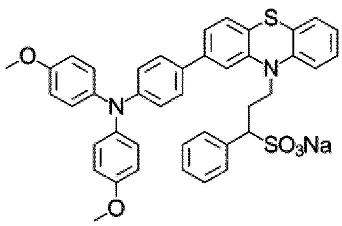

(A－43)

[Chem. 20]

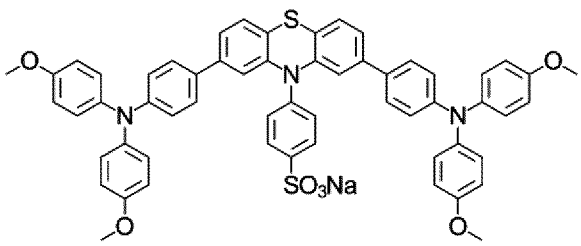

(A−44)

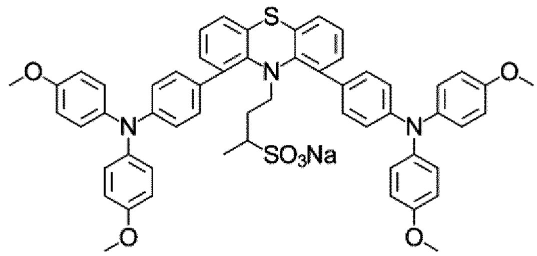

(A−45)

[Chem. 21]

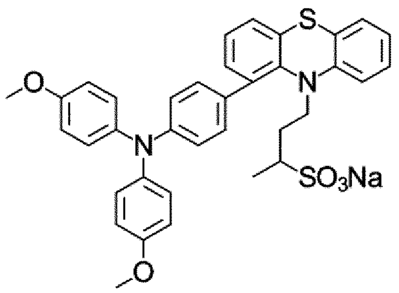

(A−46)

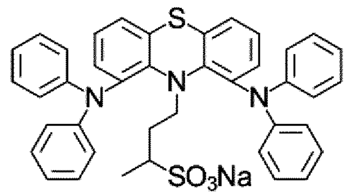

(A−47)

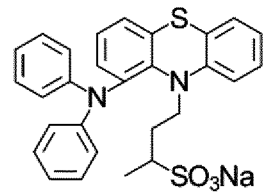

(A−48)

[Chem. 22]

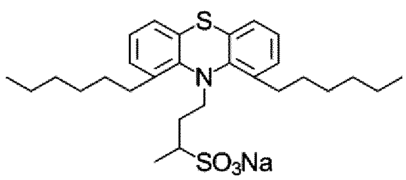

(A−49)

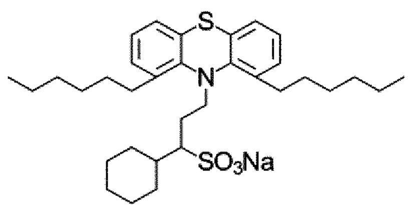

(A−50)

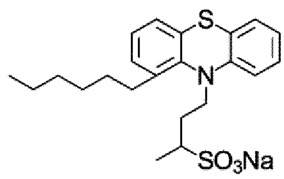

(A−51)

[Chem. 23]

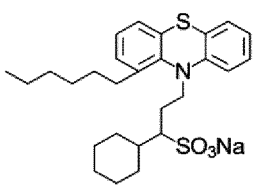

(A−52)

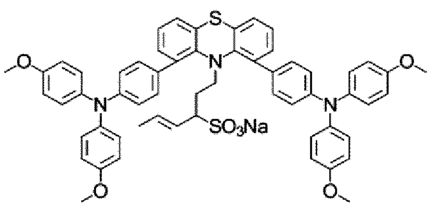

(A−53)

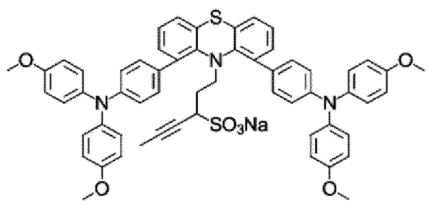

(A−54)

[Chem. 24]

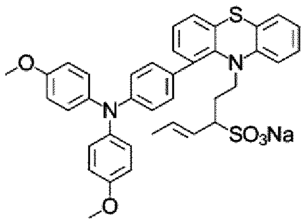

(A-55)

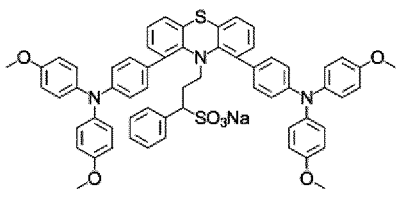

(A-56)

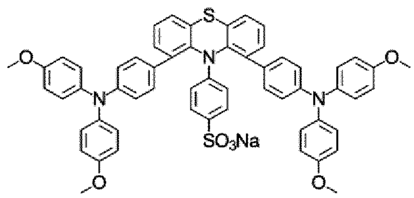

(A-57)

[Chem. 25]

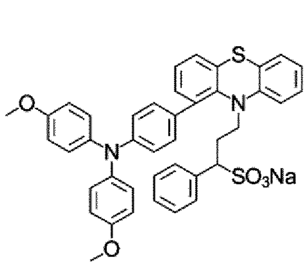

(A-58)

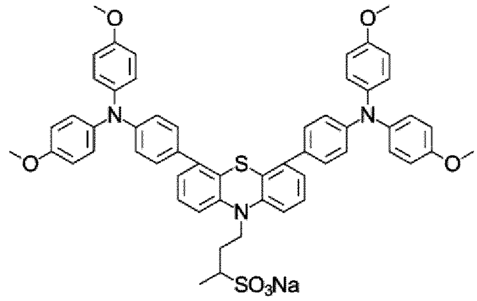

(A-59)

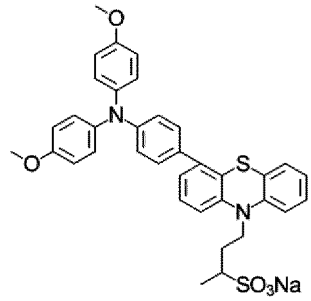

(A-60)

[Chem. 26]

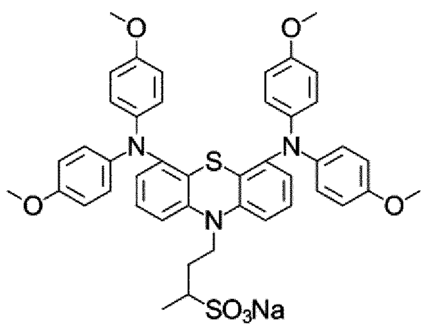

(A-61)

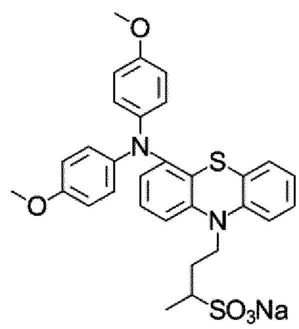

(A-62)

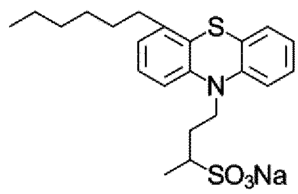

(A-63)

[Chem. 27]

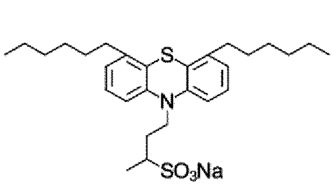

(A-64)

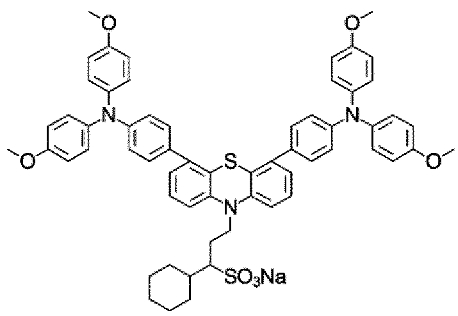

(A-65)

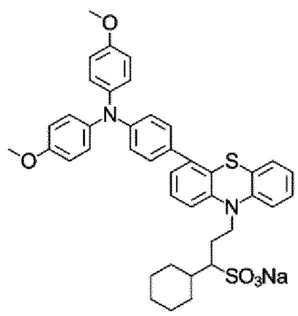

(A-66)

[Chem. 28]

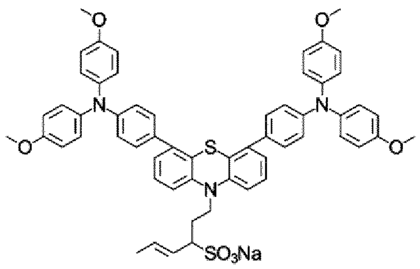
(A−67)

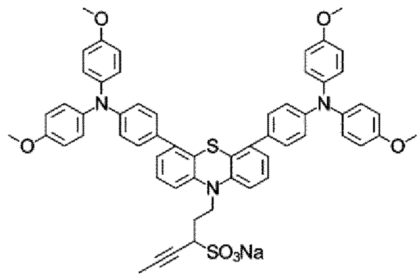
(A−68)

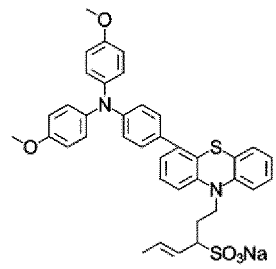
(A−69)

[Chem. 29]

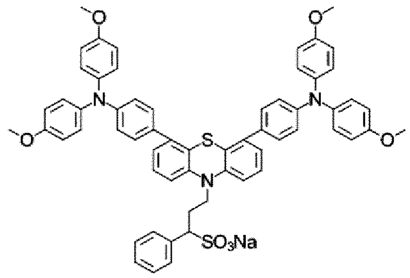
(A−70)

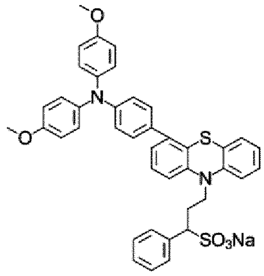
(A−71)

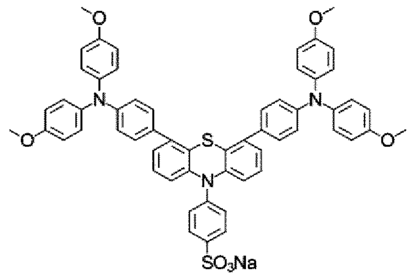
(A−72)

The compounds represented by Formula (1) of the present invention can be synthesized by a well-known method such as a method of Japanese Patent Application No. 2018-135255. A case of synthesizing a compound (A-1) will be described as an example. The compound (A-1) can be obtained by introducing a corresponding substituent to 3,7-dibromophenothiazine by a Suzuki-Miyaura coupling reaction or Buchwald reaction and then allowing the reaction product to react with a corresponding sultone. Also, the compounds represented by Formula (1) can be obtained by using a halogenated phenothiazine derivative as a precursor by a well-known method.

As a method for purifying the compounds represented by Formula (1) of the present invention, purification by column chromatography, purification by adsorption using silica gel, activated carbon, activated clay, or the like, recrystallization or crystallization with a solvent, or the like can be performed. Alternatively, it is effective to use these methods in combination to use a compound with increased purity. Identification of these compounds can be performed by nuclear magnetic resonance spectroscopy (NMR).

Each of the compounds represented by Formula (1) of the present invention can be used as a hole transport material contained in a hole transport layer for an organic electronic device such as a photoelectric conversion element and an organic EL element.

A preferred aspect of the photoelectric conversion element of the present invention is described below.

<Photoelectric Conversion Element>

The photoelectric conversion element of the present invention typically includes a conductive support 1, an electron transport layer 2, a photoelectric conversion layer 3, a hole transport layer 4, and a counter electrode 5, as shown in a schematic cross-sectional view of the FIGURE.

For the purpose of preventing dopant diffusion, a hole transport layer may be inserted between the photoelectric conversion layer 3 and the hole transport layer 4 to form a multi-layer structure that a hole transport layer is inserted.

The photoelectric conversion element of the present invention preferably includes the conductive support 1, the electron transport layer 2, the photoelectric conversion layer 3, the hole transport layer 4, and the counter electrode 5 as shown in the FIGURE, but is not limited thereto. The photoelectric conversion element of the present invention is preferably used as a solar cell, more preferably a perovskite photoelectric conversion element, but is not limited thereto. In the present invention, the perovskite photoelectric conversion element preferably includes the conductive support (electrode) 1, the electron transport layer 2, the photoelectric conversion layer (perovskite layer) 3, the hole transport layer 4, and the counter electrode 5 in this order. The perovskite photoelectric conversion element may include the conductive support, the hole transport layer, the photoelectric conversion layer (perovskite layer), the electron transport

layer, and the counter electrode in this order.

<Conductive Support>

In the photoelectric conversion element of the present invention, the conductive support 1 shown in the FIGURE must have a translucency that allows transmission of light contributing to photoelectric conversion. The conductive support is preferably a conductive substrate because the conductive support is a member having a function of extracting current from the photoelectric conversion layer. Specific examples of a conductive material may include a conductive transparent oxide semiconductor such as tin-doped indium oxide (ITO), zinc-doped indium oxide (IZO), tungsten-doped indium oxide (IWO), zinc aluminum oxide (AZO), fluorine-doped tin oxide (FTO), indium oxide ($In_2O_3$), and indium-tin composite oxide. And, tin-doped indium oxide (ITO), fluorine-doped tin oxide (FTO), and the like are preferably used.

<Electron Transport Layer>

In the photoelectric conversion element of the present invention, the electron transport layer 2 shown in the FIGURE is a layer located between the conductive support 1 and the photoelectric conversion layer (perovskite layer) 3, and the electron transport layer 2 is preferably formed on the conductive support 1. However, it is not limited to specific configurations. The electron transport layer is used to improve an efficiency of electron transfer from the photoelectric conversion layer to the electrode and to block transfer of a hole.

In the present invention, specific examples of a semiconductor forming the electron transport layer may include metal oxide such as tin oxide (SnO, SnOz, $SnO_3$, or the like), titanium oxide ($TiO_2$ or the like), tungsten oxide ($WO_2$, $WO_3$, $W_2O_3$, or the like), zinc oxide (ZnO), niobium oxide (NbzOs or the like), tantalum oxide (TazOs, or the like), yttrium oxide ($Y_2O_3$ or the like), and strontium titanate ($SrTiO_3$ or the like); metal sulfide such as titanium sulfide, zinc sulfide, zirconium sulfide, copper sulfide, tin sulfide, indium sulfide, tungsten sulfide, cadmium sulfide, and silver sulfide; metal selenide such as titanium selenide, zirconium selenide, indium selenide, and tungsten selenide; and an elemental semiconductor such as silicon and germanium, and one or more of these semiconductors are preferably used. In the present invention, one or more selected from tin oxide, titanium oxide, and zinc oxide are preferably used as the semiconductor.

In the present invention, a commercially available paste containing semiconductor fine particles may be used, or a paste prepared by dispersing a commercially available semiconductor fine powder in a solvent (coating solution for electron transport layer) may be used. Specific examples of the solvent used in preparing the paste may include water; an alcoholic solvent such as methanol, ethanol, or isopropyl alcohol; a ketone solvent such as acetone, methyl ethyl ketone, or methyl isobutyl ketone; and a hydrocarbon solvent such as n-hexane, cyclohexane, benzene, or toluene, but are not limited thereto. Also, these solvents can be used singly or as a mixed solvent of two or more.

Examples of a method for dispersing the semiconductor fine powder in the solvent in the present invention include grounding the powder in a mortar or the like and then dispersing the grounded powder, and using a dispersing machine such as a ball mill, a paint conditioner, a vertical bead mill, a horizontal bead mill, or an attritor. When the paste is prepared, a surfactant or the like is preferably added to prevent aggregation of the semiconductor fine particles, and a thickener such as polyethylene glycol is preferably added to increase viscosity.

In the present invention, the electron transport layer can be obtained by using a well-known film-forming method, depending on a material to be formed. As a film-forming method for the electron transport layer, any coating method using a coating solution can be used. Examples include a method in which a film is formed by coating a conductive substrate with a wet coating method such as a spin coating method, an inkjet method, a doctor blade method, a drop casting method, a squeegee method, a screen printing method, a reverse roll coating method, a gravure coating method, a kiss coating method, a roll brush method, a spray coating method, an air knife coating method, a wire barber coating method, a pipe doctor method, an impregnation coating method, and a curtain coating method, and then baking to remove a solvent and additives, a sputtering method, a deposition method, an electrodeposition method, an electrolyte deposition, and a microwave irradiation method, but the present invention is not limited thereto. In the present invention, it is preferable to form a film by a spin coating method using the coating solution for electron transport layer prepared by the above method, but the present invention is not limited thereto. Spin coating conditions can be set as appropriate. An atmosphere in which the film is formed is not limited, and may be in the air.

From a viewpoint of further improving the photoelectric conversion efficiency, a thickness of the electron transport layer is preferably 5 nm to 100 nm, and more preferably 10 nm to 50 nm, when a dense electron transport layer is used. In the present invention, when a porous (mesoporous) metal oxide is used in addition to the dense layer, a thickness thereof is preferably 20 nm to 200 nm, and more preferably 50 nm to 150 nm.

<Photoelectric Conversion Layer>

In the photoelectric conversion element of the present invention, the photoelectric conversion layer (perovskite layer) 3 is preferably formed on the electron transport layer 2 shown in the FIGURE.

In the present invention, when used as a perovskite photoelectric conversion element, the perovskite material that is the photoelectric conversion layer represents a series of materials having a structure represented by a Formula $ABX_3$. Here, A, B, and X each represent an organic cation or a monovalent metal cation, a metal cation, and a halide anion, and examples include A = $K^+$, $Rb^+$, $Cs^+$, $CH_3NH_3^+$ (hereinafter, MA: methylammonium), $NH{=}CHNH_2^+$ (hereinafter, FA: formamidinium), $CH_3CH_2NH_3^+$ (hereinafter, EA:ethylammonium); B = Pb, Sn; and X = $I^-$ and Br. More specifically, a layer containing a perovskite material represented by any composition of $MAPbI_3$, $FAPbI_3$, $EAPbI_3$, $CsPbI_3$, $MASnI_3$, $FASnI_3$, $EASnI_3$, $MAPbBr_3$, $FAPbBr_3$, $EAPbBr_3$, $MASnBr_3$, $FASnBr_3$, and $EASnBr_3$, and perovskite materials of a mixed cation and a mixed anion represented by any composition of $(FAMA)Pb(IBr)_3$, $K(FAMA)Pb(IBr)_3$, $Rb(FAMA)Pb(IBr)_3$, and $Cs(FAMA)Pb(IBr)_3$ can be used, but the present invention is not limited thereto. One or more of these perovskite materials are preferably used. A light absorber other than the perovskite material may be contained.

Any coating method can be used as a method for coating the photoelectric conversion layer (perovskite layer) of the photoelectric conversion element of the present invention with a coating solution, and the same method as the film-forming method for the electron transport layer can be used.

A perovskite precursor may be a commercially available material, and in the present invention, is preferably a precursor including lead halide, methylammonium halide, formamidine halide, and cesium halide with any composition, but the perovskite precursor is not limited thereto.

Examples of a solvent for a perovskite precursor solution of the present invention include N,N-dimethylformamide (DMF), dimethylsulfoxide (DMSO), and $\gamma$-butyrolactone from a viewpoint of solubility of the precursor, but the solvent is not limited thereto. These solvents may be used singly or in combination of two or more, and a mixed solution of N,N-dimethylformamide and dimethylsulfoxide is preferably used.

In the present invention, an atmosphere during film formation of the photoelectric conversion layer (perovskite layer) is preferably a dry atmosphere, and more preferably a dry inert gas atmosphere in a glove box or the like, because the atmosphare allows for preventing contamination of moisture and producing a highly efficient perovskite solar cell with good reproducibility. And during the film formation, dehydrating with a molecular sieve or the like and using a solvent with a low moisture content are preferable.

In the present invention, from a viewpoint of producing the perovskite material from the precursor, a temperature for heating the photoelectric conversion layer (perovskite layer) with a hot plate or the like is preferably 50°C to 200°C, and more preferably 70°C to 150°C. A heating time is preferably about 10 minutes to 90 minutes, and more preferably about 10 minutes to 60 minutes.

From a viewpoint of further preventing performance deterioration due to defects and peeling, and in order to ensure that the photoelectric conversion layer has a sufficient light absorption rate and an element resistance does not become too high, a thickness of the photoelectric conversion layer (perovskite layer) of the present invention is preferably 50 nm to 1000 nm, and more preferably 300 nm to 700 nm.

<Hole Transport Layer>

In the photoelectric conversion element of the present invention, the hole transport layer 4 shown in the FIGURE is a layer having a function of transporting a hole, and is a layer located between the photoelectric conversion layer (perovskite layer) 3 and the counter electrode 5. The hole transport layer is used to improve an efficiency of hole transfer from the photoelectric conversion layer to the electrode and to block transfer of the electron. The hole transport layer can use, for example, a conductor, a semiconductor, or an organic hole transport material, and may contain an additive for further improving hole transport characteristics. It is desirable to reduce an amount of the additive to be used in the hole transport layer, and the photoelectric conversion element of the present invention also exhibits excellent characteristics without the additive in the hole transport layer.

The hole transport layer of the present invention is a layer containing the compound represented by Formula (1) as the hole transport material. In the hole transport layer of the present invention, the compounds represented by Formula (1) may be used singly or in combination of two or more, and may also be used in combination with another hole transport material that does not belong to the present invention. For preventing dopant diffusion between the photoelectric conversion layer and the hole transport layer, also for forming a multi-layer structure using a layer (hereinafter, also referred to as the intermediate layer) in which the hole transport layer is inserted, the compound represented by Formula (1) can be contained in the intermediate layer, and the hole transport material that does not belong to the present invention can also be used as the hole transport layer.

Specific examples of the another hole transport material that does not belong to the hole transport material of the present invention include a compound semiconductor containing monovalent copper such as CuI, $CuInSe_2$, or CuS;

and a compound containing a metal other than copper, such as GaP, NiO, CoO, FeO, $Bi_2O_3$, $MoO_2$, or $Cr_2O_3$, and these metal oxides may be mixed in the hole transport layer or laminated on the hole transport material. Examples of the organic hole transport material include a polythiophene derivative such as poly-3-hexylthiophene (P3HT) or polyethylenedioxythiophene (PEDOT); a fluorene derivative such as 2,2',7,7'-tetrakis-(N,N-di-p-methoxyphenylamine)-9,9'-spirobifluorene (Spiro-OMeTAD); a carbazole derivative such as polyvinylcarbazole; a triphenylamine derivative such as poly[bis(4-phenyl)(2,4,6-trimethylphenyl)amine] (PTAA); a diphenylamine derivative; a polysilane derivative; and a polyaniline derivative.

Any coating method can be used as a method for coating the hole transport layer of the photoelectric conversion element of the present invention with a coating solution, and the same method as the film-forming method for the electron transport layer can be used.

In the present invention, examples of the solvent used in the coating solution for hole transport layer when forming a film include an aromatic organic solvent such as benzene, toluene, xylene, mesitylene, tetralin (1,2,3,4-tetrahydronaphthalene), monochlorobenzene (chlorobenzene), o-dichlorobenzene, m-dichlorobenzene, p-dichlorobenzene, and nitrobenzene; a halogenated alkyl organic solvent such as dichloromethane, chloroform, 1,2-dichloroethane, 1,1,2-trichloroethane, and dichloromethane; a nitrile solvent such as benzonitrile and acetonitrile; an ether solvent such as tetrahydrofuran, dioxane, diisopropyl ether, c-pentyl methyl ether, ethylene glycol dimethyl ether, ethylene glycol diethyl ether, and propylene glycol monomethyl ether; an ester solvent such as ethyl acetate and propylene glycol monomethyl ether acetate; and an alcoholic solvent such as methanol, isopropanol, n-butanol, propylene glycol, 1,3-butanediol, 1,4-butanediol, 2,3-butanediol, cyclohexanol, and 2-n-butoxyethanol, but the present invention is not limited thereto. The above solvents may be used singly or in combination of two or more, and the solvent to be used can be selected depending on the structure. In particular, the aromatic organic solvent and the halogenated alkyl organic solvent are preferably used.

In the present invention, from a viewpoint of further improving the photoelectric conversion efficiency, a film thickness of the hole transport layer is preferably 5 nm to 500 nm, and more preferably 10 nm to 250 nm. In order to prevent dopant diffusion between the photoelectric conversion layer 4 and the hole transport layer 5, even when the hole transport layer has the multi-layer structure in which the hole transport layer is inserted, a total film thickness is preferably the same.

In the present invention, an atmosphere during film formation of the hole transport layer is preferably a dry atmosphere because the atmosphere allows for producing a highly efficient perovskite solar cell with good reproducibility by preventing contamination of moisture. A dehydrated solvent with a moisture content of 10 ppm or less is preferably used.

<Additive>

In the present invention, the hole transport layer may contain a dopant (or oxidizing agent) or a basic compound (or basic additive) as an additive. Adding an additive to the hole transport layer to increase a carrier concentration of the hole transport material in the hole transport layer (doping) leads to an improvement in the conversion efficiency of the photoelectric conversion element. In the present invention, when the hole transport layer contains the dopant and the basic additive, which are additives, an amount of the additive is preferably 3.5 equivalents or less with respect to 1 equivalent of the hole transport material. On the other hand, the use of the dopant and the basic additive, which are additives, in manufacturing the photoelectric conversion element may cause processes to be complicated and costs to increase. Also, the use of the dopant and the basic additive may lead to low durability and a short life of the element, regarding the characteristics. Therefore, it is desirable that the hole transport layer does not contain a dopant or a basic additive, or that an amount of using the dopant or the basic additive is reduced. And, the photoelectric conversion element of the present invention has high characteristics even without containing a dopant or a basic additive as an additive.

In the present invention, when the dopant is contained, specific examples of the dopant may include lithium bis(trifluoromethylsulfonyl)imide (LiTFSI), bis(trifluoromethanesulfonyl)imide silver, tris(2-(1H-pyrazol-1-yl)-4-tert-butylpyridine)cobalt(III) tri[bis(trifluoromethane)sulfonimide] (FK209), $NOSbF_6$, $SbCl_5$, and $SbF_5$. In the present invention, lithium bis(trifluoromethylsulfonyl)imide (LiTFSI) is preferably used, but the present invention is not limited thereto.

In the present invention, when the dopant is used, the dopant is preferably 2.0 equivalents or less, and more preferably 0.5 equivalents or less with respect to 1 equivalent of the hole transport material contained in the hole transport layer.

In the present invention, the basic compound (basic additive) may be contained as the additive for the hole transport layer. In the present invention, when the basic compound is contained, specific examples thereof include 4-tert-butylpyridine (tBP), 2-picoline, and 2,6-lutidine. The basic compound is often used in combination with the dopant. Also in the present invention, when the dopant is used, it is desirable to use the basic compound in combination, and it is preferable to use tert-butylpyridine.

In the present invention, when the basic compound is used, the basic compound is preferably 5 equivalents or less, and more preferably 3 equivalents or less with respect to 1 equivalent of the hole transport material of the present

invention.

<Counter Electrode>

In the present invention, the counter electrode 5 shown in the FIGURE is arranged opposite to the conductive support 1 and formed on the hole transport layer 4, so that charges can be exchanged with the hole transport layer. In the photoelectric conversion element of the present invention, it is preferable to provide a metal electrode as the counter electrode on the hole transport layer 4, and an electron blocking layer made of an organic material or an inorganic compound semiconductor can be added between the hole transport layer 4 and the counter electrode 5.
In the present invention, specific examples of the material used for the counter electrode include a metal such as platinum, titanium, stainless steel, aluminum, gold, silver, nickel, magnesium, chromium, cobalt, or copper, or an alloy thereof. In the above specific examples, it is preferable to use gold, silver, or a silver alloy since they have high electrical conductivity even in a thin film. In order to be less susceptible to sulfidation or chlorination and to improve a stability of a thin film, examples of the silver alloy include a silver-gold alloy, a silver-copper alloy, a silver-palladium alloy, a silver-copper-palladium alloy, and a silver-platinum alloy.
In the present invention, the counter electrode is preferably made of a material that can be formed by a method such as vapor deposition.
When a metal electrode is used as the counter electrode, in order to obtain excellent conductivity, a film thickness thereof is preferably 10 nm or more, and more preferably 50 nm or more.
In the photoelectric conversion element of the present invention, the conductive support serves as a cathode and the counter electrode serves as an anode. Light such as sunlight is preferably applied from a conductive support side. By irradiation of sunlight or the like, the photoelectric conversion layer (perovskite layer) absorbs light and enters an excited state, and generates the electron and the hole. The electron moves through the electron transport layer and the hole moves through the hole transport layer to the electrode, thereby causing current to flow, and functioning as a photoelectric conversion element.
When evaluating performance (characteristics) of the photoelectric conversion element of the present invention, a short-circuit current density, an open-circuit voltage, Fill Factor, and a photoelectric conversion efficiency are measured. The short-circuit current density represents current per 1 $cm^2$ that flows between two terminals when an output terminal is short-circuited, and the open-circuit voltage represents a voltage between the two terminals when the output terminal is open-circuited. The Fill Factor is a value obtained by dividing a maximum output (product of current and voltage) by a product of the short-circuit current density and the open-circuit voltage and is primarily dependent on an internal resistance. The photoelectric conversion efficiency is obtained by dividing a maximum output (W) by a light intensity (W) per 1 $cm^2$ to obtained a value and multiplying the value by 100, which is expressed as a percentage.
The photoelectric conversion element of the present invention can be applied to a perovskite solar cell, various optical sensors, and the like. The photoelectric conversion element including, as the hole transport layer, the hole transport material containing the compound represented by Formula (1) serves as a cell, the required number of cells are arranged to form a module, and predetermined electrical wiring is provided, so that the perovskite solar cell of the present invention is obtained.
Although the preferred embodiment has been described above, the present invention is not limited thereto, and may be modified as appropriate without departing from the scope of the present invention.

EXAMPLES

Hereinafter, the present invention will be specifically described with reference to Examples, but the present invention is not limited to the following Examples. Compounds obtained in Synthesis Examples were identified by $^1H$-NMR (1H-NMR (nuclear magnetic resonance apparatus manufactured by JEOL Ltd., JNM-ECZ400S/L1 type)).
Hereinafter, the present invention will be specifically described with reference to Examples, but the present invention is not limited to the following Examples. Compounds obtained in Synthesis Examples were identified by $^1H$-NMR (1H-NMR (nuclear magnetic resonance apparatus manufactured by JEOL Ltd., JNM-ECZ400S/L1 type).

[Synthesis Example 1]

Synthesis of Compound (A-1)

3,61-Dibromophenothiazine (0.71 g, manufactured by TCI), [4-[bis(4-methoxyphenyl)amino]phenyl]boronic acid (1.68 g, manufactured by TCI), 2 M potassium carbonate aqueous solution (10 mL), tetrakistriphenylphosphine palladium (0.06 g, manufactured by Kanto Kagaku Co., Ltd.), and THF (30 mL) were added in a reaction vessel, followed by degassing under reduced pressure. The mixture was stirred under reflux for 10 hours, and the reaction was completed.

After the reaction solution was added to the toluene (50 mL), liquid separation was performed. An organic layer was dried over magnesium sulfate, and then the solvent was distilled off under reduced pressure. A crude product was purified with a silica gel column (toluene), and further purified with a silica gel column ($NH_2$ modification/toluene) to obtain a compound represented by the following Formula (2) as a white solid (yield: 1.12 g, yield rate: 70%).

$^1$H-NMR (400MHz, DMSO-$d_6$):δ (ppm) = 3.73-3.75 (12H), 6.74-6.68 (2H), 6.76-6.82 (4H), 6.88-6.94 (8H), 6.99-7.05 (8H), 7.12-7.28 (4H), 7.37-7.44 (4H), 8.73 (1H).

[Chem. 30]

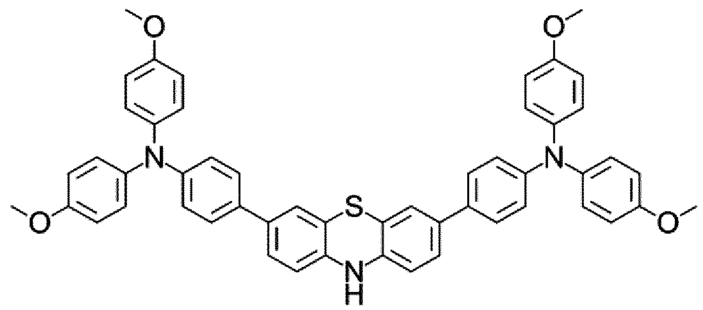

(2)

The compound (0.30 g) of the above Formula (2), sodium hydride (0.02 g, manufactured by Kanto Kagaku Co., Ltd.), and THF (12 mL) were added into a reaction vessel and the mixture was stirred at room temperature for 3 hours. To the mixture was added 2,4-butanesultone (0.063 mL, manufactured by TCI), followed by stirring for 4 hours under heating and reflux. The solvent of the reaction solution was distilled off under reduced pressure, and the obtained crude product was purified with a silica gel column (ethyl acetate). Further, purification was performed by recrystallization (acetone:ethanol) to obtain a compound represented by the following Formula (A-1) as a yellow solid (yield: 0.16 g, yield rate: 44%).

$^1$H-NMR (400MHz, DMSO-$d_6$):δ (ppm) = 1.14 (3H), 1.73 (1H), 2.21 (1H), 3.74 (12H), 3.89-4.11 (2H), 6.74-6.84 (4H), 6.87-6.97 (8H), 6.99-7.05 (8H), 7.08 (2H), 7.31-7.34 (2H), 7.38-7.42 (2H), 7.45-7.51 (4H)

[Chem. 31]

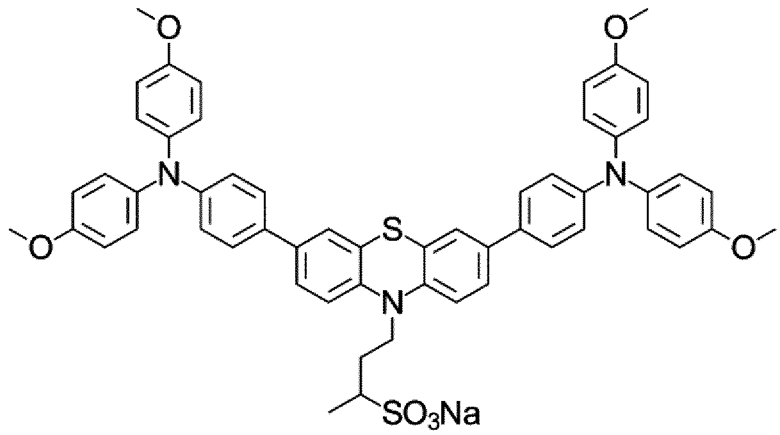

(A-1)

[Synthesis Example 2]

Synthesis of Compound (A-2)

The compound (0.20 g) of the above Formula (2), potassium tert-butoxide (0.07 g, manufactured by Kanto Kagaku Co., Ltd.), and DMF (12 mL) were added into a reaction vessel and the mixture was stirred at 60°C for 30 minutes. To the mixture was added 2,4-butanesultone (0.04 mL, manufactured by TCI), followed by heating and stirring at 60°C for 7 hours. The solvent of the reaction solution was distilled off under reduced pressure. The crude product was purified with a silica gel column (ethyl acetate/methanol) to obtain an orange oily substance (yield: 0.10 g, yield rate: 40%) according to the following Formula (A-2).

$^1$H-NMR (400MHz, DMSO-$d_6$):δ (ppm) = 1.14 (3H), 1.72 (1H), 2.17-2.22 (1H), 3.75 (12H), 3.98-4.10 (2H), 6.79

(4H), 6.91-6.97 (8H), 7.01-7.08 (10H), 7.33 (2H), 7.40 (2H), 7.46 (4H)

[Chem. 32]

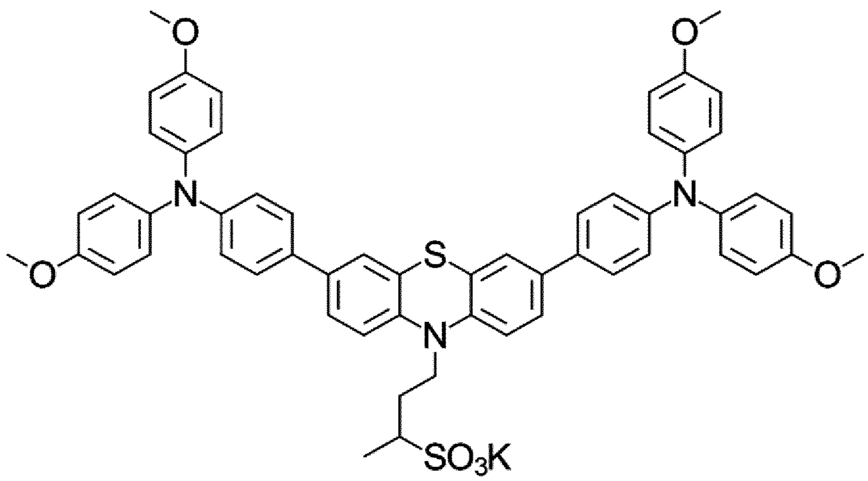

(A−2)

[Synthesis Example 3]

Synthesis of Compound (A-3)

The compound (0.40 g) of the above Formula (2), cesium carbonate (0.32 g, manufactured by Kanto Kagaku Co., Ltd.), and THF (12 mL) were added into a reaction vessel and the mixture was stirred at room temperature for 2 hours. To the mixture were added 2,4-butanesultone (0.063 mL, manufactured by TCI) and DMF (6 mL), followed by heating and stirring at 80°C for 7 hours. The reaction solution was filtered, and the solvent was distilled off from the filtrate under reduced pressure. The crude product was purified with a silica gel column (ethyl acetate/methanol), and then further purified by recrystallization (acetone:ethanol). A compound represented by the following Formula (A-3) was obtained as a yellow solid (yield: 0.01 g, yield rate: 2%).

$^1$H-NMR (400MHz, DMSO-$d_6$):$\delta$ (ppm) = 1.13 (3H), 1.72 (1H), 2.20 (1H), 3.74 (12H), 4.12 (2H), 6.79 (4H), 6.91-6.97 (8H), 7.00-(8H), 7.63 (4H), 7.69 (4H), 8.46 (2H)

[Chem. 33]

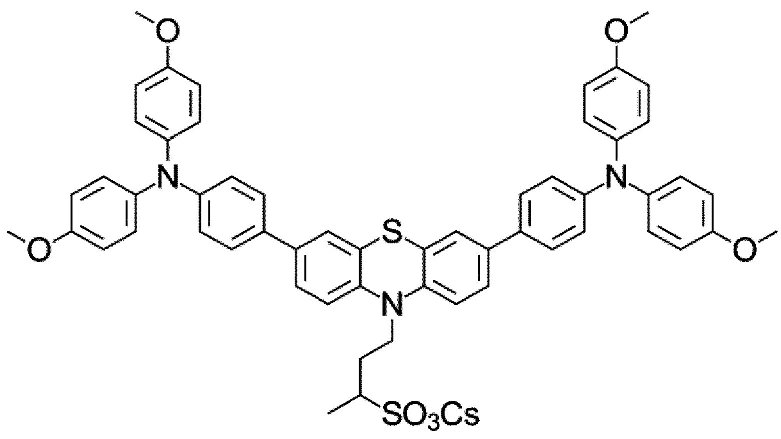

(A−3)

[Example 1]

Preparation of Photoelectric Conversion Element and Evaluation of Photoelectric Conversion Characteristics

An etched glass substrate (conductive support, manufactured by Solaronix) coated with an indium oxide and tin oxide (ITO) thin film was ultrasonically cleaned with isopropyl alcohol and then treated with UV ozone.

A tin oxide dispersion solution containing Tin (IV) oxide, 15% in $H_2O$ colloidal dispersion (manufactured by Alfa

Aesar) and purified water at a volume ratio of 1:3 was coated on this substrate by spin coating. Thereafter, an electron transport layer having a thickness of about 40 nm was formed by heating at 150°C for 30 minutes using a hot plate.

In a glove box under a nitrogen stream, formamidine hydroiodide (1 M, manufactured by Tokyo Chemical Industry Co., Ltd.), lead iodide (II) (1.1 M, manufactured by Tokyo Chemical Industry Co., Ltd.), methylamine hydrobromide (0.2 M, manufactured by Tokyo Chemical Industry Co., Ltd.), and lead bromide (II) (0.2 M, manufactured by Tokyo Chemical Industry Co., Ltd.) were dissolved in a mixed solvent of dimethylformamide and dimethylsulfoxide at a volume ratio of 4:1. A dimethylsulfoxide solution of cesium iodide (1.5 M, manufactured by Tokyo Chemical Industry Co., Ltd.) was added to the above mixture so that a charge weight of cesium was 5% in a composition ratio, and a perovskite precursor solution was prepared.

In the glove box under the nitrogen atmosphere, the prepared perovskite precursor solution was dropped and spin-coated on the tin oxide thin film, and 0.3 mL of chlorobenzene was dropped during the spin-coating to form a perovskite precursor film. Thereafter, by heating at 100°C for 1 hour using the hot plate, a $Cs(MAFA)Pb(IBr)_3$ layer (photoelectric conversion layer) having a film thickness of about 500 nm was formed.

In a glove box under a nitrogen stream, 50 mM of the compound (A-1), which is the hole transport material obtained in Synthesis Example 1, was dissolved in chlorobenzene to prepare a coating solution for hole transport layer. In the glove box under the nitrogen atmosphere, the $Cs(MAFA)Pb(IBr)_3$ layer (photoelectric conversion layer) was spin-coated with the coating solution for hole transport layer to form the hole transport layer having a thickness of about 200 nm.

A gold electrode (counter electrode 5) was provided on the hole transport layer 5 by forming a gold film in a thickness of 80 nm to 100 nm at a degree of vacuum of about $1 \times 10^{-4}$ Pa by a vacuum deposition method, thereby producing a photoelectric conversion element.

Light with an intensity of 100 $mW/cm^2$ generated by a simulated sunlight irradiation device (OTENTO-SUN III type, manufactured by Spectrometer Co., Ltd.) from a transparent electrode side of the photoelectric conversion element was emitted from the conductive support side of the photoelectric conversion element, and current-voltage characteristics were measured by using a source meter (Model 2400 General-Purpose, SourceMeter, manufactured by KEITHLEY) to determine an initial photoelectric conversion efficiency. The photoelectric conversion element was stored for 30 days from the initial photoelectric conversion efficiency measurement in a light-shielding desiccator provided with silica gel, and the current-voltage characteristics were measured again to determine a photoelectric conversion efficiency after 30 days. Table 1 shows a ratio of photoelectric conversion efficiency to Comparative Example 1, which is determined by dividing the determined photoelectric conversion efficiency after 30 days by the photoelectric conversion efficiency after 30 days of Comparative Example 1. Table 2 shows a retention rate (%) calculated from the following Formula (a-1) using the determined initial photoelectric conversion efficiency and the photoelectric conversion efficiency after 30 days, which is the over-time photoelectric conversion efficiency.

[Formula 1]

$$\text{Retention rate (\%)} = \text{Over-time photoelectric conversion efficiency} / \text{Initial photoelectric conversion efficiency} \times 100 \quad \text{(a-1)}$$

[Example 2]

Preparation of Photoelectric Conversion Element and Evaluation of Photoelectric Conversion Characteristics

Except for a compound (A-3) was used, a photoelectric conversion element was produced in the same manner as in Example 1, and the initial photoelectric conversion efficiency and the photoelectric conversion efficiency after 30 days of storage were determined in the same manner as in Example 1. Table 1 shows a ratio of photoelectric conversion efficiency to Comparative Example 1, which is determined by dividing the determined photoelectric conversion efficiency after 30 days by the photoelectric conversion efficiency after 30 days of Comparative Example 1.

[Example 3]

Preparation of Photoelectric Conversion Element and Evaluation of Photoelectric Conversion Characteristics

Except for a compound (A-2) was used, a photoelectric conversion element was produced in the same manner as in Example 1, and the initial photoelectric conversion efficiency and the photoelectric conversion efficiency after 30 days of storage were determined in the same manner as in Example 1. Table 1 shows a ratio of photoelectric conversion efficiency to Comparative Example 1, which is determined by dividing the determined photoelectric conversion efficiency after 30 days by the photoelectric conversion efficiency after 30 days of Comparative Example 1. Table 2 shows the retention rate (%) calculated from the above Formula (a-1) using the determined initial photoelectric conversion efficiency

and the over-time photoelectric conversion efficiency, which is the photoelectric conversion efficiency after 30 days.

[Comparative Example 1]

A chlorobenzene solution including 25 mM of bis(trifluoromethanesulfonyl)imide lithium (LiTFSI, 0.5 equivalents) which is a dopant of the additive and 150 mM of 4-tert-butylpyridine which is a basic compound of the additive was prepared as a doping solution. The prepared doping solution and Spiro-OMeTAD (manufactured by Sigma-Aldrich) which is a standard hole transport material represented by the following Formula (B-1) were used to prepare a 50 mM of chlorobenzene solution as the coating solution for hole transport layer. Except that the coating solution for hole transport layer was used, a photoelectric conversion element was produced in the same manner as in Example 1, and the current-voltage characteristics were measured to determine the initial photoelectric conversion efficiency and the photoelectric conversion efficiency after 30 days of storage. The photoelectric conversion efficiency after 30 days of storage was 8.08%. Table 2 shows the retention rate (%) calculated from the above Formula (a-1) using the determined initial photoelectric conversion efficiency and the photoelectric conversion efficiency after 30 days, which is the over-time photoelectric conversion efficiency.

[Chem. 34]

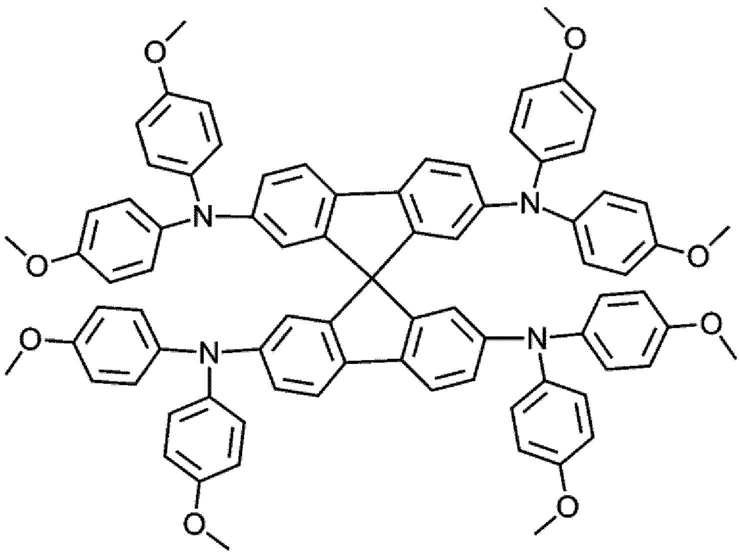

(B－1)

[Table 1]

| | Compound | Adding amount (equivalent) of dopant (LiTFSI) | Ratio of photoelectric conversion efficiency to Comparative Example 1 (after 30 days) |
|---|---|---|---|
| Example 1 | A-1 | 0 | 1.41 |
| Example 2 | A-3 | 0 | 1.27 |
| Example 3 | A-2 | 0.5 | 1.53 |
| Comparative Example 1 | B-1 | 0.5 | 1.00 |

[Table 2]

| | Compound | Adding amount (equivalent) of dopant (LiTFSI) | Retention rate (%) |
|---|---|---|---|
| Example 1 | A-1 | 0 | 136% |
| Example 3 | A-2 | 0.5 | 89% |

(continued)

| | Compound | Adding amount (equivalent) of dopant (LiTFSI) | Retention rate (%) |
|---|---|---|---|
| Comparative Example 1 | B-1 | 0.5 | 84% |

From the results in Table 1, it was found that using the hole transport layer containing the compound (A-1) or (A-3) of the present invention, even without using a dopant as an additive, achieved higher and sufficient photoelectric conversion efficiency than that of Comparative Example 1 using a comparative compound that uses a dopant as a standard. From results in Table 2, it was found that the photoelectric conversion element of Example 1 using the hole transport layer containing the compound (A-1) of the present invention without a dopant as an additive had a higher retention rate than that of the photoelectric conversion element of Comparative Example 1 using a compound (B-1) containing a dopant as an additive, and thus, a photoelectric conversion element with high durability can be produced by the compound of the present invention. From the above, the photoelectric conversion element containing the compound of the present invention can reduce manufacturing costs by not adding a dopant and a basic additive, which are additives, and can be manufactured by using a simple process that does not require addition of a dopant and a basic additive.

From the results in Table 1, it was found that the photoelectric conversion element using the compound (A-2) of the present invention as a hole transport material exhibited the excellent photoelectric conversion efficiency compared to a photoelectric conversion element using a standard hole transport material. From the results in Table 2, it was found that the photoelectric conversion element using the compound (A-2) of the present invention as a hole transport material exhibited higher durability than that of the photoelectric conversion element using the standard hole transport material.

The present application claims priority based on a Japanese Patent Application No. 2021-159732 filed on September 29, 2021.

INDUSTRIAL APPLICABILITY

A compound having a sulfonate group according to the present invention is useful for a photoelectric conversion element having good photoelectric conversion efficiency, and allows for providing clean energy as a solar cell that can efficiently convert solar energy into electrical energy, and can also be applied to an organic EL, an image sensor, and the like, when used as a hole transport layer.

**Claims**

1. A compound represented by the following Formula (1),

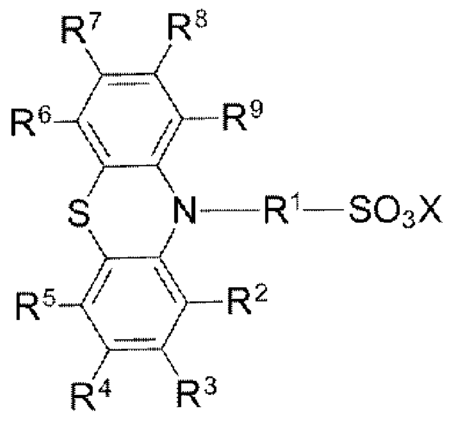

(1)

where, $R^1$ represents:

a linear or branched alkylene group having 1 to 18 carbon atoms that optionally has a substituent;
a linear or branched alkenylene group having 2 to 20 carbon atoms that optionally has a substituent;
a linear or branched alkynylene group having 2 to 20 carbon atoms that optionally has a substituent;
a cycloalkylene group having 3 to 10 carbon atoms that optionally has a substituent;
an arylene group having 6 to 36 carbon atoms that optionally has a substituent; or

a divalent heterocyclic group having 5 to 36 ring atoms that optionally has a substituent,
X represents a monovalent cation, and
$R^2$ to $R^9$ each independently represent:

a hydrogen atom;
a linear or branched alkyl group having 1 to 18 carbon atoms that optionally has a substituent;
a linear or branched alkenyl group having 2 to 20 carbon atoms that optionally has a substituent;
a linear or branched alkynyl group having 2 to 20 carbon atoms that optionally has a substituent,
a cycloalkyl group having 3 to 10 carbon atoms that optionally has a substituent;
a linear or branched alkoxy group having 1 to 20 carbon atoms that optionally has a substituent;
a cycloalkoxy group having 3 to 10 carbon atoms that optionally has a substituent;
a linear or branched alkoxycarbonyl group having 1 to 18 carbon atoms that optionally has a substituent;
a thio group having 1 to 18 carbon atoms that optionally has a substituent;
an amino group having 1 to 20 carbon atoms that optionally has a substituent;
an aromatic hydrocarbon group having 6 to 36 carbon atoms that optionally has a substituent; or
a monovalent heterocyclic group having 5 to 36 ring atoms that optionally has a substituent.

2. The compound according to claim 1, wherein in Formula (1), $R^1$ represents the linear or branched alkylene group having 1 to 18 carbon atoms that optionally has a substituent.

3. The compound according to claim 1, wherein in Formula (1), at least one of $R^2$ to $R^9$ represents the aromatic hydrocarbon group having 6 to 36 carbon atoms that optionally has a substituent or the amino group having 1 to 20 carbon atoms that optionally has a substituent.

4. The compound according to claim 3, wherein in Formula (1), at least one of $R^4$ and $R^7$ represents the aromatic hydrocarbon group having 6 to 36 carbon atoms that optionally has a substituent or the amino group having 1 to 20 carbon atoms that optionally has a substituent.

5. The compound according to claim 1, wherein in Formula (1), X represents an alkali metal ion or an ammonium ion that optionally has a substituent.

6. The compound according to claim 5, wherein in Formula (1), the alkali metal ion comprises a sodium ion, a potassium ion, a rubidium ion, or a cesium ion.

7. A hole transport material, comprising the compound according to any one of claims 1 to 6.

8. A hole transport material composition for a photoelectric conversion element, comprising the hole transport material according to claim 7.

9. A photoelectric conversion element using the hole transport material composition for photoelectric conversion according to claim 8.

10. A solar cell using the photoelectric conversion element according to claim 9.

# FIGURE

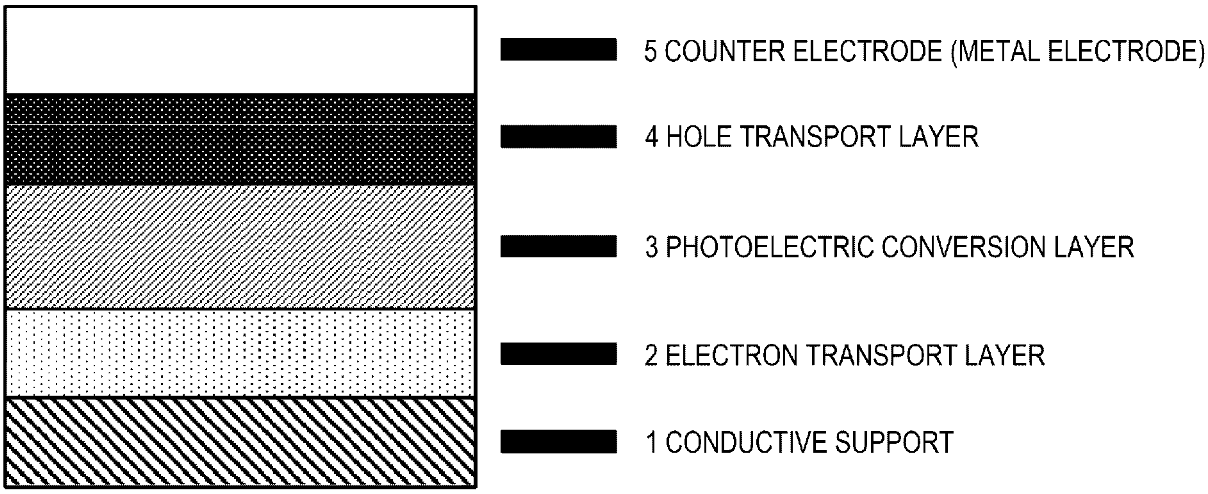

5 COUNTER ELECTRODE (METAL ELECTRODE)
4 HOLE TRANSPORT LAYER
3 PHOTOELECTRIC CONVERSION LAYER
2 ELECTRON TRANSPORT LAYER
1 CONDUCTIVE SUPPORT

**INTERNATIONAL SEARCH REPORT**

International application No. **PCT/JP2022/036015**

**A. CLASSIFICATION OF SUBJECT MATTER**

***H10K 30/50***(2023.01)i; ***C07D 279/22***(2006.01)i
FI: H01L31/04 168; H01L31/04 154B; H01L31/04 154C; H01L31/04 154D; C07D279/22; H01L31/04 112Z

According to International Patent Classification (IPC) or to both national classification and IPC

**B. FIELDS SEARCHED**

Minimum documentation searched (classification system followed by classification symbols)

H01L51/42-51/56; H01L27/30-27/32

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2014/080378 A1 (UNIVERSITY OF CAPE TOWN) 30 May 2014 (2014-05-30) p. 14, lines 1-17 | 1-2, 5-6 |
| A | | 3-4, 7-10 |
| X | US 10000643 B1 (THE UNITED STATES OF AMERICA AS REPRESENTED BY THE SECRETARY OF THE NAVY) 19 June 2018 (2018-06-19) column 4, lines 5-56 | 1-2, 5-6 |
| A | | 3-4, 7-10 |
| A | JP 2014-84282 A (SAMSUNG R&D INSTITUTE JAPAN CO LTD) 12 May 2014 (2014-05-12) | 1-10 |
| A | JP 2018-18967 A (NISSAN CHEMICAL IND LTD) 01 February 2018 (2018-02-01) | 1-10 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/036015**

| Patent document cited in search report | Publication date (day/month/year) | Patent family member(s) | Publication date (day/month/year) |
|---|---|---|---|
| WO 2014/080378 A1 | 30 May 2014 | CN 104812394 A | |
| US 10000643 B1 | 19 June 2018 | (Family: none) | |
| JP 2014-84282 A | 12 May 2014 | (Family: none) | |
| JP 2018-18967 A | 01 February 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10937972 B
- JP 2018135255 A
- JP 2021159732 A

### Non-patent literature cited in the description

- *Journal of the American Chemical Society,* 2009, vol. 131, 6050-6051
- *Science,* 2012, vol. 388, 643-647
- *Chem. Sci.,* 2019, vol. 10, 6748-6769
- *Adv. Funct. Mater.,* 2019, 1901296